# EUROPEAN PATENT APPLICATION

(11) **EP 4 772 212 A2**
(43) Date of publication of application: **08.07.2026**
(21) Application number: 26179159.4
(22) Date of filing: 01.07.2022
(51) Int. Cl.: A61M 5/142

(54) **MEDICAL DEVICE WITH ADAPTIVE CORRECTION FOR UNCONTROLLABLE DELIVERY CONDITIONS**

(30) Priority: 09.07.2021 US 202163220414 P
(62) Divisional of application: 22182585.4
(71) Applicant: CareFusion 303, Inc., San Diego, CA 92130 (US)
(72) Inventor: Ooi, Chun Keat, San Diego, 92130 (US)
(74) Representative: Zenz Patentanwälte Partnerschaft mbB

(57) **Abstract**

An infusion device is configured to monitor delivery conditions associated with a medication infusion, identify an infusion set used with the infusion device, determine that the delivery conditions modify operation of infusion set, and determine correction factors for modifying an operation of the device based on a modification of the operation of the infusion set by the monitored delivery conditions. According to various implementations, an operating parameter of the infusion device may be modified, including modifying a threshold for when the infusion device will experience an alarm condition.

## Description

### TECHNICAL FIELD

This application relates generally to maintaining the safety and performance of medical devices throughout a healthcare organization.

### BACKGROUND

Temperature and other environmental factors may influence the operation of a medical device. For example, these and other environmental conditions can interfere with the desired operation of sensitive circuitry within a medical device such as an infusion pump. These factors may have an influence on how an infusion pump delivers a medication or provides alarms. Failure of a medical device to deliver an accurate amount of medication or to alarm when appropriate can impact the patients or clinicians of a healthcare facility.

### SUMMARY

According to various aspects, the subject technology provides a machine-implemented method comprising monitoring a delivery condition associated with an infusion pump or a medication during or prior to an administration of the medication to a patient by the infusion pump; receiving an identification of an infusion set associated with the administration of the medication; determining, based on the identification of the infusion set and the monitored delivery condition, an operating characteristic representative of how the infusion set operates differently in view of the monitored delivery condition than under normal operating conditions; identifying an operating parameter programmed to the infusion pump to administer the medication to the patient and which requires adjustment in view of the operating characteristic; and automatically adjusting the identified operating parameter based on the determined operating characteristic representative of how the infusion set operates differently in view of the monitored delivery condition than under normal operating conditions. Other aspects include corresponding systems, apparatuses, and computer program products for implementation of the machine-implemented method.

According to various aspects, the subject technology provides an infusion device configured to monitor a delivery condition associated with the infusion device or a medication during or prior to an administration of the medication to a patient by the infusion device; receive an identification of an infusion set associated with the administration of the medication; determine, based on the identification of the infusion set and the monitored delivery condition, an operating characteristic representative of how the infusion set operates differently in view of the monitored delivery condition than under normal operating conditions; identify an operating parameter programmed to the infusion pump to administer the medication to the patient and which is requires adjustment in view of the operating characteristic; and automatically adjust the identified operating parameter based on the determined operating characteristic representative of how the infusion set operates differently in view of the monitored condition than under normal operating conditions. Other aspects include corresponding systems, methods, and computer program products for implementation of the features of the infusion device.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description of Implementations below, in conjunction with the following drawings. Like reference numerals refer to corresponding parts throughout the figures and description.
FIG. 1 depicts an example medical device, including one or more sensors used to assess the operating condition of the device and associated accessories and parameters in view of external factors that may affect the device's operations, according to various aspects of the subject technology.
FIG. 2 depicts an example adjustment to flow rate for a smart infusion pump, according to various aspects of the subject technology.
FIG. 3 depicts various examples of using adaptive correction to adjust an infusion site to avoid a false alarm, according to various aspects of the subject technology.
FIG. 4 depicts an example process for adaptively correcting an operational parameter of a medical device to account for uncontrollable factors, according to aspects of the subject technology.
FIG. 5 depicts an example of a patient care device such as an infusion device, according to aspects of the subject technology.
FIG. 6 is a conceptual diagram illustrating an example electronic system for automatically adjusting a parameter of a medical device responsive to one or more delivery conditions, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

Medical devices are expected to operate within certain operational tolerances. However, environmental conditions, characteristics of accessories used with the medical device, as well as patient characteristics can affect device operations, leading to operations outside these tolerances, or lead to situations in which the operational tolerances are exceeded in circumstances in which they otherwise would not. The subject technology includes a medical device monitoring and adjustment system that utilizes sensors and operational and patient data to dynamically vary the operational performance of the medical device to maintain operation within normal conditions. According to some implementations, the system may automatically make or suggest corrective action(s) to address the conditions.

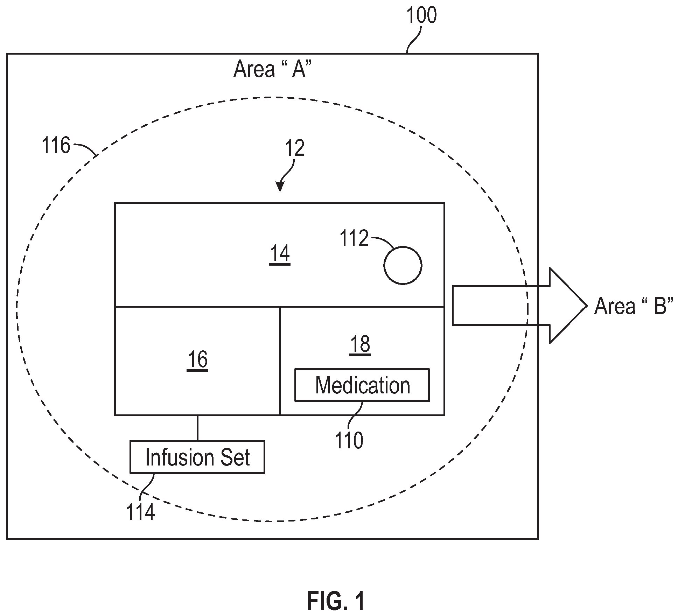

FIG. 1 depicts an example medical device 12, including one or more sensors 112 used to assess the operating condition of the device and associated accessories and parameters in view of external factors that may affect the device's operations, according to various aspects of the subject technology. While the subject technology is described with regard to a large volume infusion pump, the subject technology may be applied to numerous other pumps that utilize compression of tubing to affect the pumping action.

As shown, an infusion device 12 may be positioned in a patient care area 100, and may include a control unit 14 and one or more functional modules (e.g., functional modules 16, 18, 20, 22 of FIG. 1), including a first functional module 16 and a second functional module 18. The first functional module may include an infusion pump 16, such as a large volume infusion pump or a syringe pump. The second functional module 18 is depicted as including a medication 110. The one or more sensors 112 may be disposed within a housing of the infusion pump, or externally provided. Infusion pump 16 may be connected to a patient via an intravenous infusion line of an infusion set 114. According to various implementations, the infusion device 12 may be configured to administer the medication 110 to the patient (e.g., intravenously by way of a connected infusion set) using the infusion pump module 16, through the infusion set 114. Control unit may include one or more processors such as to be configured to interface with the functional units and to control and provide power to the functional units when the functional units are connected to the control unit.

Infusions provided by large volume pumps, for example, have performance requirements, including volume rate accuracy, air-inline detection, and occlusion alarms. Falling outside of performance requirements can lead to clinical concerns like medication efficacy and patient safety. Performance of a pump depends on various factors, including some within the control of pump/infusion set manufacturer and some not. Controllable factors include, for example, pumping mechanism accuracy, set tubing consistency (within pumping/sensor mechanism) and air/pressure sensor sensitivity. Factors generally outside direct manufacturer control include environmental conditions (e.g., temperature/pressure/humidity), flow resistance of a set type selected for that particular infusion (e.g., in-line restrictions such as filters and valves), fluid viscosity (e.g., infusate type/concentration or infusate temperature), fluid container pressure upstream of pump (e.g., based on height or container vacuum), a back pressure downstream of pump (e.g., catheter gauge or infusion site height).

The subject technology minimizes performance degradation despite variations in factors generally outside direct manufacturer control. In this regard, an infusion device is configured to monitor delivery conditions associated with a medication infusion, identify an infusion set used with the infusion device, determine that the delivery conditions modify operation of infusion set, and determine correction factors for modifying an operation of the device based on a modification of the operation of the infusion set by the monitored delivery conditions. According to various implementations, an operating parameter of the infusion device may be modified. In some implementations, the operating parameter includes a threshold for when the infusion device will experience an alarm condition. Various implementations are proposed for correcting different parameters and/or for determining and/or characterizing operational conditions for correction of one or more respective parameters. Each of the implementations disclosed herein can be used individually or in combination, depending on which factors has the most significant effect on which important performance requirement.

According to various aspects of the subject technology, the infusion device 12 may be configured to monitor, using sensor(s) 112, an environmental condition of a physical environment proximate to a medical device. In this regard, sensor(s) 112 may include a thermistor, radio frequency (RF) receiver, magnetometer, light sensor, and the like. For example, a sensor 112 may be configured to detect ambient temperature, radiant temperature, an amount of light, acoustical transmissions (sound), a magnetic field, or strength of a magnetic field. Sensor(s) 112 may have a range 114. Additionally or in the alternative, as will be described further, sensors 112 may include one or more internal sensors for measuring internal conditions of the device 12, or may include sensors for measuring operational conditions of other medical devices and/or accessories utilized during the operation of the device 12.

According to various implementations, the device 12 may include and/or utilize one or more on-board sensors 112 such as a magnetometer, temperature sensor, acoustic sensor, or light sensor, that are configured to measure internal conditions of the device. An internal sensor 112 may measure cam rotation. The device 12 may include circuitry and/or software that monitors the measurements and/or values received from each of these sensors. When a device (or its network circuitry) 12 detects one of these measurements and/or values satisfies (e.g., exceeds) of affects a predetermined threshold or tolerance, the medical device may be triggered to automatically adjust the corresponding parameter or change modes.

When receiving a measurement value, medical device 12 may determine whether the value affects or potentially affects operation by performing calculations to determine whether the device, operating under the measured condition, would operate outside an expected tolerance or threshold with regard to care of a patient. In this manner, the infusion device 12 may preemptively adjust certain parameters to avoid the potential for faulty operation such as providing an incorrect dosage to failing to provide alarms or alerts in an appropriate manner.

Various thresholds may, for example, be stored in a database and indexed by one or more indexes including care area 100, patient medical condition, time of day, and the like. Responsive to the value exceeding the threshold, an operational parameter can be adjusted automatically to perform normally under the measured condition(s), or an operation mode of the device 12 may be automatically switched from a first mode currently programmed for the care of the patient to a second mode. According to various aspects, the second mode may utilize different parameters than the first mode to control the medical device. For example, a first mode may operate a pump according to a first flow rate and/or pumping speed, and a second mode may operate the pump according to a second, lower flow rate and/or pumping speed. In some implementations, the second mode may be a different pumping speed to achieve a predetermined or user programmed flow rate under changed conditions. A parameter of a first mode may activate a functional module, and parameter of a second mode may deactivate the functional module.

The device 12 may be configured to scan a medication and/or an infusion set used in a current administration for the patient, and consider the qualities or characteristics of the medication and/or infusion set, and how they interact with each other, in view of other measured conditions (e.g., environmental conditions) when determining whether an operational parameter or hardware mechanism of the device 12 should be adjusted. Medication and infusion set characteristics, as well as any associated operational thresholds and ranges disclosed herein, may be stored, for example, in a lookup table or databased indexed by corresponding identifiers. For example, an infusion device may lookup safe temperatures for a scanned medication and automatically measure temperature values that may affect the medication. An infusion device may, on being programmed to administer a given medication, lookup ranges for environmental conditions such as temperature and light for the given medication and/or infusion set delivering the medication, and begin monitoring environmental conditions proximate to the device to ensure these ranges are satisfied. When a medical device (or its network circuitry) detects one of these measurements and/or values would affect the infusion with regard to the infusion device, the medication or the infusion set, the medical device may be triggered to automatically adjust a parameter or change modes so that the infusion remains unaffected.

Each infusion set and medication for which data is stored may be first subjected to lab/bench testing to estimate/characterize effect size/magnitude of one or more predetermined uncontrollable factors on each performance requirement. For example, an infusion set may be subjected to testing to determine normal operations and conditions, and how normal operation of an infusion set changes with respect to different medication types, including how each medication changes its flow under different environmental conditions such as ambient temperature. For the purpose of this disclosure normal operations and/or conditions may include those parameters and/or environmental conditions used during manufacturer testing and characterization process to set a baseline for any variations and/or changes observed during the testing. Normal operations and/or conditions may also be referred to as, for example, a default operation and/or condition.

Further characterizations may include how flow is changed when each infusion set is used with a different size catheter. In this regard, an identifier may be associated with each infusion set, catheter, and medication, and potential changes may be determined based on indexing the lookup table based on the various indexes. In this manner, a suitable correction factor may be calculated after the characterization, the correction factor indexed by device and medication identifiers, and operational performance of the infusion device, such as a pumping mechanism speed/pressure sensor alarm threshold, can be adjusted accordingly by pump software during infusion based on a lookup of the stored correction factors.

To minimize performance degradation from variation in environmental conditions, temperature/pressure/humidity sensors 112 within the infusion device 12 are used to periodically measure environmental conditions surrounding the infusion device and generate a suitable correction factor to achieve a nominal operational status of the infusion device. For example, when an infusion is started, the clinician may provide an identification of an infusion set associated with the administration of the medication. In some implementations, the infusion set may be scanned by a scanner and the identification passed to the infusion device 12 by way of the scan. For example, the clinician may scan an infusion set label (e.g., a barcode on packaging) before use, and flow resistance for that set is fed back to pump for the suitable correction factor. The infusion device 12 is configured with sensors 12 and measures an environmental condition during or prior to the administration of the medication to the patient by the infusion pump.

Based on the identification of the infusion set and the measured delivery condition, the infusion device 12 determines an operating characteristic of the infusion set corresponding to the measured environmental condition. For example, the infusion device 12 may index the previously described lookup table using the identification of the infusion set and determine the previously stored characterization information for the measured environmental condition. As an example, the characterization may inform the infusion device that an infusion threshold (e.g., a tolerance) for one or more operating parameters is affected when an intravenous infusion line of the infusion is under the measured environmental condition. The infusion device may then adjust the identified operating parameter so that the threshold is satisfied in accordance with the infusion set operating under normal delivery conditions (e.g., different than the measured condition).

The foregoing adjustment may be made in consideration of other delivery conditions, in addition to or as an alternative to environmental conditions. For example, the infusion device may determine a real-time delivery pressure associated with the administration of the medication to the patient, perform a lookup to determine an infusion characterization parameter based on the real-time delivery pressure, the operating characteristic of the infusion set, and the measured environmental condition, and adjust the operating parameter based on the operating characteristic of the infusion set comprises adjusting the operation parameter to account for a pressure variance in the infusion line due to the medication being infused through the infusion set at the environmental condition. The operating parameter may be, for example, a pump speed or a flow rate of the medication within an intravenous tube of the infusion set.

As another example, the characterizations may be used to minimize performance degradation from variations in set flow resistance. In this regard, the flow resistance of all infusion sets compatible with the infusion device 12 are characterized. As described previously, the characterizations may further be cross indexed with other characterizations such as medications and temperatures to obtain a correction factor for an operating parameters such as pump speed and/or flow rate and/or pressure. A sticky infusate, for example, may not perform well with certain smaller catheters or filters, and may perform worse when temperatures are varied beyond a particular threshold.

Characterizations may further be made based on patient physiological parameters. For example, the patient's, height, weight, or blood pressure may affect the flow resistance or pressure or what size of catheter should be used as to not adversely affect the flow resistance or pressure.

Characterizations may also similarly be used to minimize performance degradation from a variation in fluid viscosity. In this regard, the viscosities of commonly-used infusate type/concentration (and infusate temperature if typically stored in fridge before use) are characterized. The infusate type/concentration identified by the infusion device (or monitoring system), for example, by a clinician inputting the identification into pump before infusion, or by barcode scanning of pre-filled infusate container, or through remote database connection for clinician's drug prescription, etc.). The characterization may be further cross referenced with other characterizations, as previously described, and suitable correction factor can be determined and applied.

A degradation in performance may also be inferred from a variation in fluid container height or the possibility of a vacuum. In some implementations, height or vacuum identification may be identified through user input into the device 12. In some implementations, a pressure sensor within the pump can be used to measure pressure upstream of pumping mechanism. The characterization data may then be cross referenced with other data, as previously described, and a suitable correction factor determined and applied.

As another example, the characterizations may be used to minimize performance degradation from backpressure. In some implementations, a pressure sensor 112 within the infusion device 12 can be used to measure pressure downstream of the pumping mechanism. In some implementations, back pressure may be derived from external subfactors. For example, backpressure may be determined based on pump height, height of the infusate container, catheter size, flow resistance of the identified infusion set, whether any filters are used and the type of filter, the height of the infusion site with respect to the pump or container, and the like. These subfactors may be received by the infusion device prior to or during operation by way of user input by a clinician.

The determined backpressure may be used to determine characterization data which may then be cross referenced with other data, as previously described, and a suitable correction factor determined and applied. For example, an increased backpressure of X may lead to a decreased infusion of Y (e.g., a 10% decrease over a predetermined period of time). The correction factor may be applied to the pump's flow rate or pumping speed, thereby increasing the rate to accommodate for the decrease of Y. For example, the correction factor may cause the pump to operate 5-10% faster than it would without detection of the backpressure.

According to various implementations, the foregoing characterizations are evaluated holistically to arrive at a correction factor. Each characterization (e.g., of a temperature, fluid characteristic, infusion set type, etc.) is considered with regard to how the characterization may affect each operating parameter of the infusion device (e.g., flow rate, pressure, pump speed, etc.) or other characterizations. The correction factor is then derived in real-time to account for any deviation from an expected (e.g., normal or tested) operating performance of the infusion device under normal conditions.

The subject technology's contribution of characterizations on demand and responsiveness to dynamically changing real-time conditions mitigates or even eliminates many problems in existing solutions, including achieving consistent alarm detections and/or flow rate accuracy throughout a lifetime of a device and/or throughout a patient's hospitalization.

Upon receiving identification data (e.g., of devices, infusion sets, or medications), the characterization data are input into an algorithm to determine the adjustments to be made. The algorithm may further function as a machine learning algorithms and revise estimations based on further input or real-time results and conditions. Computer program code for carrying out operations of the subject technology may be written in an object oriented programming language such as, for example, JAVA^{®}, Smalltalk, or C++. However, the computer program code for carrying out operations of the subject technology may also be written in conventional procedural programming languages, such as the "C" programming language, in an interpreted scripting language, such as Perl, or in a functional (or fourth generation) programming language such as Lisp, SML, Forth, or the like. The software may also be written to be compatible with HLA-7 requirements.

With further reference to FIG. 1, according to various implementations, adjusting an operational parameter may include adjusting a threshold for an alarm or alert by the device 12. In some implementations, a medical device 12 may be configured to produce audible or visual alerts when a threshold is reached. The alert may include providing for display an option for adjusting a parameter. For example, the medical device may prompt a user to select whether to maintain a current operational parameter or confirm a new updated parameter based on the foregoing characterizations.

An operating parameter affected by environmental and other external conditions, as described previously, may include a threshold for providing the alarm. Accordingly, characterization data may inform the device 12 that the alarm may be affected, and to adjust the threshold so that any alarm operates as expected. For example, the device 12 may determine that operation of the infusion set under a measured delivery condition (e.g., temperature, pressure, viscosity, etc.) affects how the threshold is satisfied for the administration of the medication to the patient. As one example, the threshold may be triggered earlier due to an incorrect flow rate reading. If the flow rate is indicated higher than it really is, a flow alarm be issued prematurely. Use of a fine filter with certain heavier or sticky infusates may cause a false occlusion alarm. A viscosity of a medication that is outside of an expected viscosity due to temperature or other factors may further affect the alarm. The identified operating parameter (e.g., flow rate, viscosity) may be adjusted based on the characterizations so that the threshold is satisfied in accordance with the infusion set operating under a normal delivery condition different than the measured delivery condition.

In some implementations, under an alert condition the device may be prevented from administering or providing further medications until the alert has been acknowledged. Acknowledgement may include identifying a clinician authorized to the medical device by way of the clinician scanning a badge, and the clinician manually dismissing the alert by way of a manual input at the medical device or by way of a computing device connected to the medical device (e.g., over a network). Accordingly, if the parameter affecting the alert is adjusted and/or corrected, the alarm may be prevented and the device not locked.

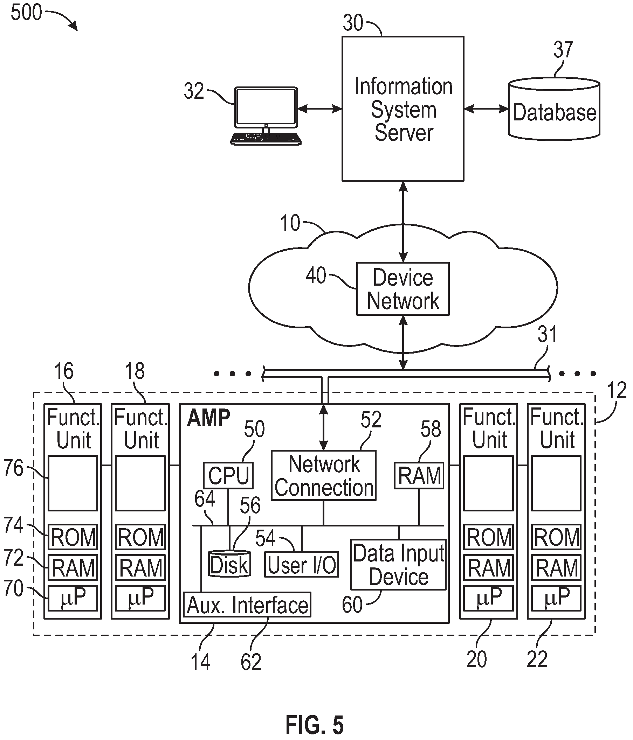

The device 12 may include circuitry that causes sensor(s) 112 to obtain a measurement values representative various conditions. Sensor(s) 112 may perform measurements periodically, or upon certain trigger conditions. One example trigger condition may include the device 12 detecting that it has moved to a new location, for example, from Area "A" to Area "B". With brief reference to FIG. 5, device 12 may query an internal database 56 or a server 30 and/or a corresponding external database 37 based on its known coordinates (e.g., GPS) or based on connecting to a new WiFi system to determine whether its new physical environment includes different or changed conditions that may affect the operational performance of the device 12. In this regard, device 12 may monitor the conditions periodically irrespective of its position, or be triggered to obtain (e.g., additional) measurements upon detecting it has been moved to a new location. For example, trigger actions may include an internal GPS sensor of the device 12 detecting the device has moved or has moved outside of a predetermined area, or the device 12 detects it has joined a new (e.g., WiFi) network node, or its current WiFi signal has degraded.

Accordingly, the infusion device may measure a delivery condition (such as delivery line pressure, temperature, humidity, etc.) at one location and then measure a changed delivery condition at a new location. The infusion device 12 may then determine, based on the identification of the infusion set and the changed delivery condition, an updated operating characteristic of the infusion set corresponding to the changed delivery condition, and automatically adjust the identified operating parameter based on the updated operating characteristic of the infusion set corresponding to the changed delivery condition.

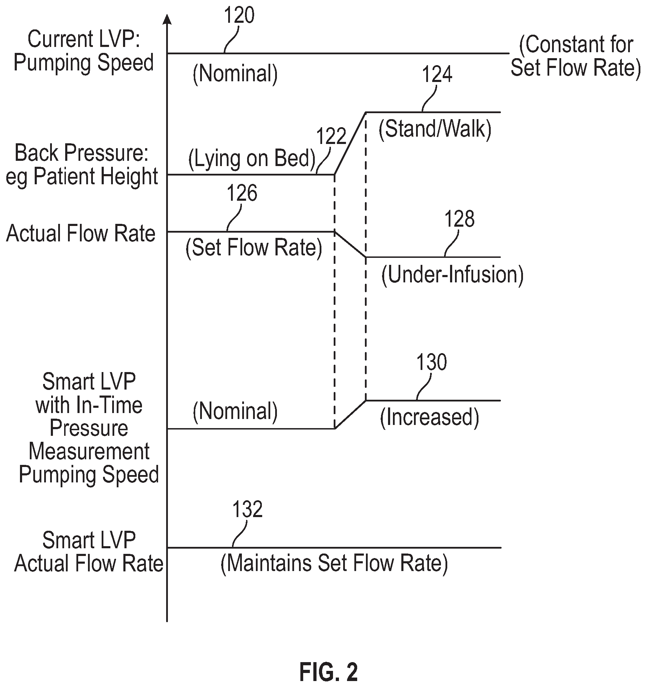

FIG. 2 depicts an example adjustment to pumping speed for a smart infusion pump, according to various aspects of the subject technology. The top row depicts of a constant pumping speed (120) of a large volume infusion pump. The pumping speed remains constant once flow rate is programmed by user, despite external factors which may influence the ongoing infusion. For example, the patient may be lying on a bed (nominal back pressure) when the pump is programed (122), but then chose to stand or walk leading to increased back pressure (124). The actual flow rate is initially equal to set flow rate (126), but may then decrease due to increased back pressure (128), as depicted in the example of FIG. 2, thereby leading to an eventual under-infusion. However, a "smart" pump, according to the subject technology, may adjust the nominal rate according to external factors. Here, the pump increases (130) the pumping speed on detecting that back pressure has increased e.g., due to the height of the catheter (and patient) increased. The flow rate may be adjusted for the duration corresponding to the changed circumstance or condition. Even though the pump's pumping speed is increased, it is increased to maintain the actual flow rate (132) it would have had, had the default condition remained (e.g., had the patient not elected to stand). When the default condition returns (e.g., the patient returns to bed) or a different condition presents itself, the pump may again adjust pumping speed to maintain the originally desired actual flow rate, despite the changed conditions.

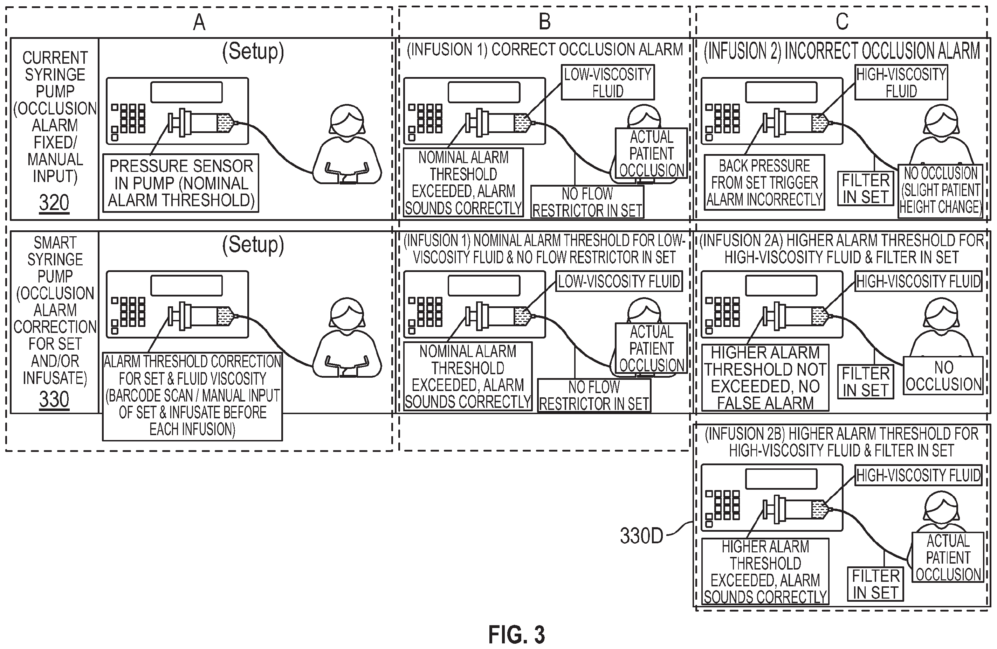

FIG. 3 depicts various examples of using adaptive correction to adjust an infusion site to avoid a false alarm, according to various aspects of the subject technology. A first example 320 depicts two scenarios in which a current pump implementation without the subject technology. First, a syringe pump is setup to detect occlusions using a pressure sensor based on a nominal alarm threshold (320A) intended for nominal delivery conditions (low-viscosity fluid and set without flow restrictor). In the first scenario (320B), a low viscosity fluid is infused into a patient, with no flow restrictor in place. Under these nominal circumstances, when an actual patient occlusion occurs, an occlusion alarm sounds correctly. In the second scenario (320C), a high viscosity fluid is infused into the same patient, with a flow restrictor in place. This gives a significant back pressure without actual patient occlusion, and the occlusion alarm sounds incorrectly because the pump does not detect or correct for a deviation from nominal delivery conditions.

A second example 330 depicts the syringe pump setup according to the subject technology. In the first scenario (330B), the low viscosity fluid is again infused into the patient, with no flow restrictor in place. Under these circumstances, when an actual patient occlusion occurs, the occlusion alarm (at nominal threshold) sounds correctly. In the second scenario (330C), a high viscosity fluid is infused into the same patient, with a flow restrictor in place. Under these circumstances, with the subject technology, the occlusion alarm threshold is changed due to the change from nominal delivery conditions (in terms of set restriction & fluid viscosity), and the occlusion alarm correctly does not sound because the pump detects/corrects for the deviation from nominal delivery conditions. There are no false alarms. When an actual occlusion does occur (330D), the alarm sounds correctly because the infusion satisfied a corrected threshold (e.g., a higher alarm threshold).

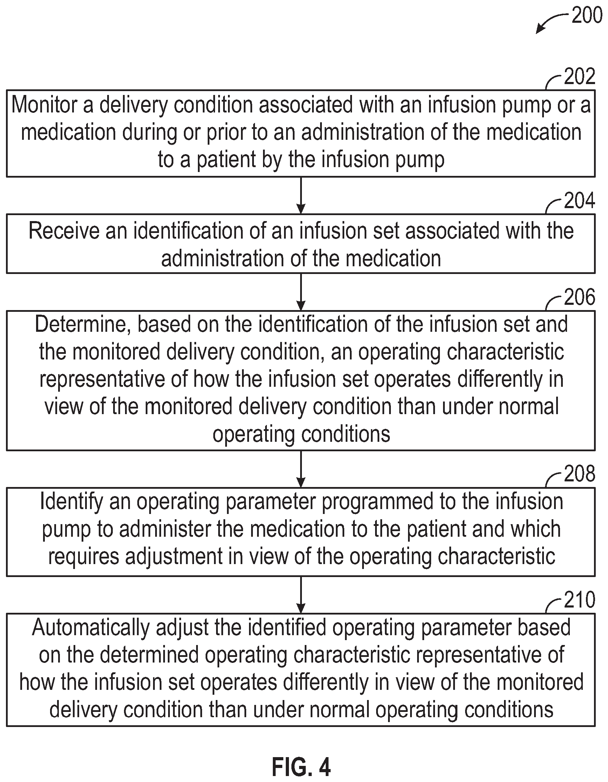

FIG. 4 depicts an example process for adaptively correcting an operational parameter of a medical device to account for uncontrollable factors, according to aspects of the subject technology. For explanatory purposes, the various blocks of example process 200 are described herein with reference to FIGS. 1-3, 5, and 6, and the components and/or processes described herein. The one or more of the blocks of process 200 may be implemented, for example, by one or more computing devices including, for example, medical device 12. In some implementations, one or more of the blocks may be implemented based on one or more machine learning algorithms. In some implementations, one or more of the blocks may be implemented apart from other blocks, and by one or more different processors or devices. Further for explanatory purposes, the blocks of example process 200 are described as occurring in serial, or linearly. However, multiple blocks of example process 200 may occur in parallel. In addition, the blocks of example process 200 need not be performed in the order shown and/or one or more of the blocks of example process 200 need not be performed.

In the depicted example, a medical device 12 monitors, various delivery conditions associated with the administration of a medication to the patient. In this regard, the medical device 12 receives one or more inputs from a clinician regarding the setup of the infusion. These inputs may include operational parameters from which the delivery conditions may be derived, including an identification of the infusion set used, type of medication and/or a medication order, and various physiological parameters of the patient (e.g., height, weight, blood pressure). One or more of these parameters may be manually entered at a user interface of the device. One or more of these parameters may be scanned. And, one or more of these features may be automatically measured by the device 12. For example, the device may automatically detect a temperature or humidity of an area 114 proximate to the device 12. Once the infusion set is loaded, the infusion device 12 may automatically detect other parameters such as an infusion line set size and a flow rate and/or pressure of the medication.

In the depicted example, the infusion device 12 receives (e.g., by way of monitoring) a delivery condition associated with an infusion pump or a medication during or prior to an administration of the medication to a patient by the infusion pump (202). As described previously, the delivery condition may be received by way of an entered or measured parameter. The infusion device 12 also receives an identification of an infusion set associated with the administration of the medication to be used in the infusion (204). The infusion device 12 then determines, based on the identification of the infusion set and the received delivery condition, an operating characteristic of the infusion set corresponding to the received delivery condition (206). As described previously, the infusion device 12 may perform a lookup based on the received and/or measured identifications to obtain various characterization data associated with the identifications.

The infusion pump identifies (from or based on the entered data), an operating parameter programmed to the infusion pump to administer the medication to the patient and which requires adjustment according to the delivery condition and the infusion set used during the administration of the medication (208). In some implementations, the operating parameter may be predetermined to require adjustment according to the delivery condition and the infusion set. For example, a lookup table may be indexed by delivery condition and/or infusion set identifiers to determine operating parameter(s) that require adjustment, and may provide the specific adjustments to the operation based on the respective delivery condition and/or type of infusion set. The infusion device 12 then automatically adjusts the identified operating parameter based on the determined operating characteristic of the infusion set corresponding to the received delivery condition (210).

According to some implementations, when the delivery condition is an environmental condition, the infusion device 12 may further determine a real-time delivery pressure associated with the administration of the medication to the patient. Determining the real-time delivery pressure may include measuring an upstream line pressure of the intravenous tube of the infusion set. Determining the real-time delivery pressure may include measuring a downstream line pressure of the intravenous tube. In some implementations, parameters received may include a catheter size of a catheter used in the administration of the medication. In such instances, determining the real-time delivery pressure may include estimating a fluid pressure within an intravenous tube of the infusion set based on the operating characteristic of the infusion set and the received catheter size of the catheter used in the administration of the medication. The pressure may be further estimated based on the received patient physiological parameter.

In some implementations, the operating parameter to be adjusted may be a pump speed or a flow rate of the medication within an intravenous tube of the infusion set. In this regard, adjusting the operating parameter based on the operating characteristic of the infusion set may include adjusting the operation parameter to account for a pressure variance in the infusion line due to the medication being infused through the infusion set at the environmental condition. A temperature of an area proximate to the infusion pump or of the medication, may be determined to affect a fluid viscosity of the medication. Accordingly, the correction factor may be applied to correct for the difference between normal operation and the same operation involving the affected viscosity.

According to various implementations, the operating parameter may be modified based on a recalibration or recharacterization of a delivery parameter associated with the infusion set. For example, pumping speed may be calculated to achieve set flow rate without consideration to significant flow restrictors or high-viscosity fluids within an infusion set. When the infusion set is identified by the device 12 as being affected by one or more conditions, a correction factor may be applied in the calculation to correct for the affected infusion set. For example, the correction factor may be applied to correct for how the infusion set reacts to different temperatures or humidity and/or medication types. In some implementations, the corrected parameter (e.g., flow rate) may be displayed to a user. In some implementations, the corrected parameter may only be used internally by the device with regard to alarms and notifications. Where the operating parameter is a threshold for providing an alarm, the infusion device 12 may determine that an operation of the infusion set under the measured delivery condition affects how the threshold is satisfied for the administration of the medication to the patient, and adjust the identified operating parameter so that the threshold is satisfied in accordance with the infusion set operating under a normal delivery condition different than the measured delivery condition.

Many of the above-described example 200, and related features and applications, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

FIG. 5 depicts an example of a patient care device 500 such as an infusion device, according to aspects of the subject technology. In FIG. 5, the patient care device (or "medical device" generally) 12 is connected to a hospital network 10. The term patient care device (or "PCD") may be used interchangeably with the term patient care unit (or "PCU"), either which may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices. Each element 12 is connected to an internal healthcare network 10 by a transmission channel 31. Transmission channel 31 is any wired or wireless transmission channel, for example an 802.11 wireless local area network (LAN). In some implementations, network 10 also includes computer systems located in various departments throughout a hospital. For example, network 10 of FIG. 1 optionally includes computer systems associated with an admissions department, a billing department, a biomedical engineering department, a clinical laboratory, a central supply department, one or more unit station computers and/or a medical decision support system. As described further below, network 10 may include discrete subnetworks. In the depicted example, network 10 includes a device network 40 by which patient care devices 12 (and other devices) communicate in accordance with normal operations.

Additionally, the overall system of the infusion device 500 may incorporate a separate information system server 30, the function of which will be described in more detail below. Moreover, although the information system server 30 is shown as a separate server, the functions and programming of the information system server 30 may be incorporated into another computer, if such is desired by engineers designing the institution's information system. Institutional patient care system 300 may further include one or multiple device terminals 32 for connecting and communicating with information system server 30. Device terminals 32 may include personal computers, personal data assistances, mobile devices such as laptops, tablet computers, augmented reality devices, or smartphones, configured with software for communications with information system server 30 via network 10.

Patient care device 12 may include or incorporate pumps, physiological monitors (e.g., heart rate, blood pressure, ECG, EEG, pulse oximeter, and other patient monitors), therapy devices, and other drug delivery devices may be utilized according to the teachings set forth herein. In the depicted example, patient care device 12 comprises a control module 14, also referred to as interface unit 14, connected to one or more functional modules 16, 18, 20, 22. Interface unit 14 includes a central processing unit (CPU) 50 connected to a memory, for example, random access memory (RAM) 58, and one or more interface devices such as user interface device 54, a coded data input device 60, a network connection 52, and an auxiliary interface 62 for communicating with additional modules or devices. Interface unit 14 also, although not necessarily, includes a main non-volatile storage unit 56, such as a hard disk drive or non-volatile flash memory, for storing software and data and one or more internal buses 64 for interconnecting the aforementioned elements.

In various implementations, user interface device 54 is a touch screen for displaying information to a user and allowing a user to input information by touching defined areas of the screen. Additionally or in the alternative, user interface device 54 could include any means for displaying and inputting information, such as a monitor, a printer, a keyboard, softkeys, a mouse, a track ball and/or a light pen. Data input device 60 may be a bar code reader capable of scanning and interpreting data printed in bar coded format. Additionally or in the alternative, data input device 60 can be any device for entering coded data into a computer, such as a device(s) for reading a magnetic strips, radio-frequency identification (RFID) devices whereby digital data encoded in RFID tags or smart labels (defined below) are captured by the reader 60 via radio waves, PCMCIA smart cards, radio frequency cards, memory sticks, CDs, DVDs, or any other analog or digital storage media. Other examples of data input device 60 include a voice activation or recognition device or a portable personal data assistant (PDA). Depending upon the types of interface devices used, user interface device 54 and data input device 60 may be the same device. Although data input device 60 is shown in FIG. 1 to be disposed within interface unit 14, it is recognized that data input device 60 may be integral within pharmacy system 34 or located externally and communicating with pharmacy system 34 through an RS-232 serial interface or any other appropriate communication means. Auxiliary interface 62 may be an RS-232 communications interface, however any other means for communicating with a peripheral device such as a printer, patient monitor, infusion pump or other medical device may be used without departing from the subject technology. Additionally, data input device 60 may be a separate functional module, such as modules 16, 18, 20 and 22, and configured to communicate with controller 14, or any other system on the network, using suitable programming and communication protocols.

Network connection 52 may be a wired or wireless connection, such as by Ethernet, WiFi, BLUETOOTH, an integrated services digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 16, 18, 20, 22 are any devices for providing care to a patient or for monitoring patient condition. As shown in FIG. 1, at least one of functional modules 16, 18, 20, 22 may be an infusion pump module such as an intravenous infusion pump for delivering medication or other fluid to a patient. For the purposes of this discussion, functional module 16 is an infusion pump module. Each of functional modules 18, 20, 22 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor, or an intracranial pressure monitor or the like. Functional module 18, 20 and/or 22 may be a printer, scanner, bar code reader or any other peripheral input, output or input/output device.

Each functional module 16, 18, 20, 22 communicates directly or indirectly with interface unit 14, with interface unit 14 providing overall monitoring and control of device 12. Functional modules 16, 18, 20, 22 may be connected physically and electronically in serial fashion to one or both ends of interface unit 14 as shown in FIG. 1, or as detailed in Eggers et al. However, it is recognized that there are other means for connecting functional modules with the interface unit that may be utilized without departing from the subject technology. It will also be appreciated that devices such as pumps or patient monitoring devices that provide sufficient programmability and connectivity may be capable of operating as stand-alone devices and may communicate directly with the network without connected through a separate interface unit or control unit 14. As described above, additional medical devices or peripheral devices may be connected to patient care device 12 through one or more auxiliary interfaces 62.

Each functional module 16, 18, 20, 22 may include module-specific components 76, a microprocessor 70, a volatile memory 72 and a nonvolatile memory 74 for storing information. It should be noted that while four functional modules are shown in FIG. 1, any number of devices may be connected directly or indirectly to central controller 14. The number and type of functional modules described herein are intended to be illustrative, and in no way limit the scope of the subject technology. Module-specific components 76 include any components necessary for operation of a particular module, such as a pumping mechanism for infusion pump module 16.

While each functional module may be capable of a least some level of independent operation, interface unit 14 monitors and controls overall operation of device 12. For example, as will be described in more detail below, interface unit 14 provides programming instructions to the functional modules 16, 18, 20, 22 and monitors the status of each module.

Patient care device 12 is capable of operating in several different modes, or personalities, with each personality defined by a configuration database. The configuration database may be a database 56 internal to patient care device, or an external database 37. A particular configuration database is selected based, at least in part, by patient-specific information such as patient location, age, physical characteristics, or medical characteristics. Medical characteristics include, but are not limited to, patient diagnosis, treatment prescription, medical history, medical records, patient care provider identification, physiological characteristics or psychological characteristics. As used herein, patient-specific information also includes care provider information (e.g., physician identification) or a patient care device's 10 location in the hospital or hospital computer network. Patient care information may be entered through interface device 52, 54, 60 or 62, and may originate from anywhere in network 10, such as, for example, from a pharmacy server, admissions server, laboratory server, and the like.

Medical devices incorporating aspects of the subject technology may be equipped with a Network Interface Module (NIM), allowing the medical device to participate as a node in a network. While for purposes of clarity the subject technology will be described as operating in an Ethernet network environment using the Internet Protocol (IP), it is understood that concepts of the subject technology are equally applicable in other network environments, and such environments are intended to be within the scope of the subject technology.

Data to and from the various data sources can be converted into network-compatible data with existing technology, and movement of the information between the medical device and network can be accomplished by a variety of means. For example, patient care device 12 and network 10 may communicate via automated interaction, manual interaction or a combination of both automated and manual interaction. Automated interaction may be continuous or intermittent and may occur through direct network connection 54 (as shown in FIG. 1), or through RS232 links, MIB systems, RF links such as BLUETOOTH, IR links, WLANS, digital cable systems, telephone modems or other wired or wireless communication means. Manual interaction between patient care device 12 and network 10 involves physically transferring, intermittently or periodically, data between systems using, for example, user interface device 54, coded data input device 60, bar codes, computer disks, portable data assistants, memory cards, or any other media for storing data. The communication means in various aspects is bidirectional with access to data from as many points of the distributed data sources as possible. Decision-making can occur at a variety of places within network 10. For example, and not by way of limitation, decisions can be made in HIS server 30, decision support 48, remote data server 49, hospital department or unit stations 46, or within patient care device 12 itself.

All direct communications with medical devices operating on a network in accordance with the subject technology may be performed through information system server 30, known as the remote data server (RDS). In accordance with aspects of the subject technology, network interface modules incorporated into medical devices such as, for example, infusion pumps or vital signs measurement devices, ignore all network traffic that does not originate from an authenticated RDS. The primary responsibilities of the RDS of the subject technology are to track the location and status of all networked medical devices that have NIMs, and maintain open communication

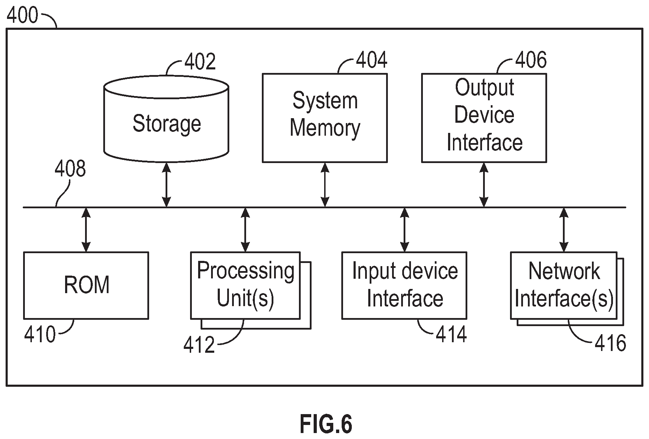

FIG. 6 is a conceptual diagram illustrating an example electronic system 400 for automatically adjusting a parameter of a medical device responsive to one or more delivery conditions, according to aspects of the subject technology. Electronic system 400 may be a computing device for execution of software associated with one or more portions or steps of process 200, or components and processes provided by FIGS. 1-3, including but not limited to information system server 30, computing hardware within patient care device 12, or terminal device 32. Electronic system 400 may be representative, in combination with the disclosure regarding FIGS. 1-5. In this regard, electronic system 400 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 400 may include various types of computer readable media and interfaces for various other types of computer readable media. In the depicted example, electronic system 400 includes a bus 408, processing unit(s) 412, a system memory 404, a read-only memory (ROM) 410, a permanent storage device 402, an input device interface 614, an output device interface 406, and one or more network interfaces 416. In some implementations, electronic system 400 may include or be integrated with other computing devices or circuitry for operation of the various components and processes previously described.

Bus 408 collectively represents all system, peripheral, and chipset buses that communicatively connect the numerous internal devices of electronic system 400. For instance, bus 408 communicatively connects processing unit(s) 412 with ROM 410, system memory 404, and permanent storage device 402.

From these various memory units, processing unit(s) 412 retrieves instructions to execute and data to process in order to execute the processes of the subject disclosure. The processing unit(s) can be a single processor or a multi-core processor in different implementations.

ROM 410 stores static data and instructions that are needed by processing unit(s) 412 and other modules of the electronic system. Permanent storage device 402, on the other hand, is a read-and-write memory device. This device is a non-volatile memory unit that stores instructions and data even when electronic system 400 is off. Some implementations of the subject disclosure use a mass-storage device (such as a magnetic or optical disk and its corresponding disk drive) as permanent storage device 402.

Other implementations use a removable storage device (such as a floppy disk, flash drive, and its corresponding disk drive) as permanent storage device 402. Like permanent storage device 402, system memory 404 is a read-and-write memory device. However, unlike storage device 402, system memory 404 is a volatile read-and-write memory, such a random access memory. System memory 404 stores some of the instructions and data that the processor needs at runtime. In some implementations, the processes of the subject disclosure are stored in system memory 404, permanent storage device 402, and/or ROM 410. From these various memory units, processing unit(s) 412 retrieves instructions to execute and data to process in order to execute the processes of some implementations.

Bus 408 also connects to input and output device interfaces 414 and 406. Input device interface 414 enables the user to communicate information and select commands to the electronic system. Input devices used with input device interface 414 include, e.g., alphanumeric keyboards and pointing devices (also called "cursor control devices"). Output device interfaces 406 enables, e.g., the display of images generated by the electronic system 400. Output devices used with output device interface 406 include, e.g., printers and display devices, such as cathode ray tubes (CRT) or liquid crystal displays (LCD). Some implementations include devices such as a touchscreen that functions as both input and output devices.

Also, as shown in FIG. 6, bus 408 also couples electronic system 400 to a network (not shown) through network interfaces 416. Network interfaces 416 may include, e.g., a wireless access point (e.g., Bluetooth or WiFi) or radio circuitry for connecting to a wireless access point. Network interfaces 416 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 400 can be used in conjunction with the subject disclosure.

These functions described above can be implemented in computer software, firmware or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra-density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an inter-network (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

### Illustration of Subject Technology as Clauses:

Various examples of aspects of the disclosure are described as numbered clauses (1, 2, 3, etc.) for convenience. These are provided as examples, and do not limit the subject technology. Identifications of the figures and reference numbers are provided below merely as examples and for illustrative purposes, and the clauses are not limited by those identification
Clause 1. A method, comprising: monitoring a delivery condition associated with an infusion pump or a medication during or prior to an administration of the medication to a patient by the infusion pump; receiving an identification of an infusion set associated with the administration of the medication; determining, based on the identification of the infusion set and the monitored delivery condition, an operating characteristic representative of how the infusion set operates differently in view of the monitored delivery condition than under normal operating conditions; identifying an operating parameter programmed to the infusion pump to administer the medication to the patient and which is requires adjustment in view of the operating characteristic; and automatically adjusting the identified operating parameter based on the determined operating characteristic representative of how the infusion set operates differently in view of the monitored delivery condition than under normal operating conditions.
Clause 2. The method of Clause 1, wherein the delivery condition is an environmental condition, the method further comprising: determining a real-time delivery pressure associated with the administration of the medication to the patient; and determining an infusion characterization parameter based on the real-time delivery pressure, the operating characteristic of the infusion set, and the measured environmental condition; wherein the operating parameter is a pump speed or a flow rate of the medication within an intravenous tube of the infusion set, wherein adjusting the operating parameter based on the operating characteristic of the infusion set comprises adjusting the operation parameter to account for a pressure variance in the infusion line due to the medication being infused through the infusion set at the environmental condition.
Clause 3. The method of Clause 2, wherein determining the real-time delivery pressure comprises measuring an upstream line pressure of the intravenous tube of the infusion set.
Clause 4. The method of Clause 2 or Clause 3, further comprising: receiving a catheter size of a catheter used in the administration of the medication, wherein determining the real-time delivery pressure comprises estimating a fluid pressure within an intravenous tube of the infusion set based on the operating characteristic of the infusion set and the received catheter size of the catheter used in the administration of the medication.
Clause 5. The method of Clause 4, further comprising: receiving a patient physiological parameter for the patient, wherein the estimating of the fluid pressure is further based on the patient physiological parameter.
Clause 6. The method of any one of Clauses 2 through 5, further comprising: receiving a medication identifier for the medication; determining, based on medication identifier, a medication characteristic of the medication; wherein the operating characteristic of the infusion set is also determined based on the identified medication characteristic.
Clause 7. The method of Clause 6, wherein the delivery condition comprises a temperature of an area proximate to the infusion pump or of the medication, and wherein the medication characteristic comprises a fluid viscosity at the temperature.
Clause 8. The method of any one of Clauses 1 through 7, wherein the delivery condition comprises an ambient temperature or humidity of an area proximate to the infusion pump, a temperature of the medication, and wherein the operating characteristic of the infusion set is based on a performance characterization of the infusion set at the temperature or humidity.
Clause 9. The method of any one of Clauses 1 through 8, wherein the delivery condition comprises a circuit temperature associated with circuitry within the infusion pump.
Clause 10. The method of any one of Clauses 1 through 9, wherein the operating parameter is a threshold for providing an alarm regarding the administration of the medication to the patient by the infusion pump, the method further comprising: determining that operation of the infusion set under the monitored delivery condition affects how the threshold is satisfied for the administration of the medication to the patient; and adjusting the identified operating parameter so that the threshold is satisfied in accordance with the infusion set operating under a delivery condition different than the monitored delivery condition.
Clause 11. The method any one of Clauses 1 through 10, further comprising, after monitoring the delivery condition and the administration of the medication has started: measuring a changed delivery condition; determining, based on the identification of the infusion set and the changed delivery condition, an updated operating characteristic of the infusion set corresponding to the changed delivery condition; automatically adjusting the identified operating parameter based on the updated operating characteristic of the infusion set corresponding to the changed delivery condition.
Clause 12. An infusion system comprising: a sensor configured to monitor a delivery condition associated with the infusion device or a medication during or prior to an administration of the medication to a patient by an infusion device; and an infusion device comprising at least one processor and a non-transitory computer readable medium storing instructions that, when executed by the at least one processor, cause the infusion device to: receive an identification of an infusion set associated with the administration of the medication; determine, based on the identification of the infusion set and the monitored delivery condition, an operating characteristic representative of how the infusion set operates differently in view of the monitored delivery condition than under normal operating conditions; identify an operating parameter programmed to the infusion pump to administer the medication to the patient and which requires adjustment in view of the operating characteristic; and automatically adjust the identified operating parameter based on the determined operating characteristic representative of how the infusion set operates differently in view of the monitored delivery condition than under normal operating conditions.
Clause 13. The infusion system of Clause 12, wherein the infusion device comprises a housing including a fluid path to receive at least a portion of the infusion set, wherein the sensor is disposed within the housing, wherein the delivery condition is an environmental condition within the housing of the infusion device, and wherein the non-transitory computer readable medium stores instructions that, when executed by the at least one processor, cause the infusion device to: determine a real-time delivery pressure associated with the administration of the medication to the patient; and determine an infusion characterization parameter based on the real-time delivery pressure, the operating characteristic of the infusion set, and the measured environmental condition; wherein the operating parameter is a pump speed or a flow rate of the medication within an intravenous tube of the infusion set, wherein adjusting the operating parameter based on the operating characteristic of the infusion set comprises adjusting the operation parameter to account for a pressure variance in the infusion line due to the medication being infused through the infusion set at the environmental condition.
Clause 14. The infusion system of Clause 13, wherein the instructions further cause the infusion device to: receive a catheter size of a catheter used in the administration of the medication, wherein determining the real-time delivery pressure comprises estimating a fluid pressure within an intravenous tube of the infusion set based on the operating characteristic of the infusion set and the received catheter size of the catheter used in the administration of the medication.
Clause 15. The infusion system of Clause 14, wherein the instructions further cause the infusion device to: receive a patient physiological parameter for the patient, wherein the estimating of the fluid pressure is further based on the patient physiological parameter.
Clause 16. The infusion device of any one of Clauses 13 through 15, wherein the instructions further cause the infusion device to: receive a medication identifier for the medication; determine, based on medication identifier, a medication characteristic of the medication; wherein the operating characteristic of the infusion set is also determined based on the identified medication characteristic.
Clause 17. The infusion device of Clause 16, wherein the delivery condition comprises a temperature of an area proximate to the infusion device or of the medication, and wherein the medication characteristic comprises a fluid viscosity at the temperature.
Clause 18. The infusion device of any one of Clauses 12 through 18, wherein the delivery condition comprises an ambient temperature or humidity of an area proximate to the infusion device, a temperature of the medication, and wherein the operating characteristic of the infusion set is based on a performance characterization of the infusion set at the temperature or humidity.
Clause 19. The infusion device of any one of Clauses 12 through 19, wherein the operating parameter is a threshold for providing an alarm regarding the administration of the medication to the patient by the infusion pump, wherein the instructions further cause the infusion device to: determine that operation of the infusion set under the monitored delivery condition affects how the threshold is satisfied for the administration of the medication to the patient; and adjust the identified operating parameter so that the threshold is satisfied in accordance with the infusion set operating under a normal delivery condition different than the monitored delivery condition.
Clause 20. A non-transitory computer readable medium having instructions stored thereon that, when executed by a medical device, cause the medical device to: monitor a delivery condition associated with the infusion pump or a medication during or prior to an administration of the medication to a patient by the infusion pump; receive an identification of an infusion set associated with the administration of the medication; determine, based on the identification of the infusion set and the monitored delivery condition, an operating characteristic representative of how the infusion set operates differently in view of the monitored delivery condition than under normal operating conditions; identify an operating parameter programmed to the infusion pump to administer the medication to the patient and which requires adjustment in view of the operating characteristic; and automatically adjust the identified operating parameter based on the determined operating characteristic representative of how the infusion set operates differently in view of the monitored delivery condition than under normal operating conditions.

### Further consideration:

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The term website, as used herein, may include any aspect of a website, including one or more web pages, one or more servers used to host or store web related content, etc. Accordingly, the term website may be used interchangeably with the terms web page and server. The predicate words "configured to", "operable to", and "programmed to" do not imply any particular tangible or intangible modification of a subject, but, rather, are intended to be used interchangeably. For example, a processor configured to monitor and control an operation or a component may also mean the processor being programmed to monitor and control the operation or the processor being operable to monitor and control the operation. Likewise, a processor configured to execute code can be construed as a processor programmed to execute code or operable to execute code.

Features described may include machine learning. Machine learning may include models, equations, artificial neural networks, recurrent neural networks, convolutional neural networks, decision trees, or other machine readable artificial intelligence structure. Examples of machine learning and modeling features which may be included in the embodiments discussed above are described in "A survey of machine learning for big data processing" by Qiu et al. in EURASIP Journal on Advances in Signal Processing (2016) which is hereby incorporated by reference in its entirety.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

As used herein, the terms "real time" "realtime" or "real time" generally signify a time frame for the associated concept. For example, real time processing of an input refers to a process that receives the input and provides a response without observable latency during the process. In contrast, a non-real time processing of the input may include storing the input for assessment at a later time (e.g., according to a schedule).

## Claims

1. A method, comprising:
monitoring an environmental condition associated with an infusion pump or a medication during or prior to an administration of the medication by the infusion pump;
receiving an identification of an infusion set associated with the administration of the medication;
determining, based on the identification of the infusion set and the monitored environmental condition, an operating characteristic that is representative of how the infusion set operates differently in view of the monitored environmental condition than under normal operating conditions;
identifying a pump speed or a flow rate of a medication programmed to the infusion pump to administer the medication and which requires adjustment in view of the operating characteristic; and
automatically adjusting the pump speed or the flow rate to account for a pressure variance in an infusion line of the infusion set due to the medication being infused through the infusion set at the monitored environmental condition.

2. The method of Claim 1, further comprising:
determining a real-time delivery pressure associated with the administration of the medication; and
determining a correction factor based on the real-time delivery pressure, the operating characteristic of the infusion set, and the monitored environmental condition, wherein automatically adjusting the pump speed or the flow rate comprises applying the correction factor to the pump speed or the flow rate to account for the pressure variance in the infusion line.

3. The method of Claim 2, wherein determining the real-time delivery pressure comprises measuring an upstream line pressure of the infusion line of the infusion set.

4. The method of Claim 2 or Claim 3, further comprising:
receiving a catheter size of a catheter used in the administration of the medication, wherein determining the real-time delivery pressure comprises estimating a fluid pressure within the infusion line of the infusion set based on the operating characteristic of the infusion set and the received catheter size of the catheter used in the administration of the medication.

5. The method of Claim 4, further comprising:
receiving a patient physiological parameter for a patient, wherein the estimating of the fluid pressure is further based on the patient physiological parameter.

6. The method of any one of Claims 1-5, wherein the operating characteristic comprises at least one of (i) a backpressure associated with the infusion set or (ii) a flow resistance of the infusion set, and wherein the pressure variance in the infusion line is due to the backpressure and/or the flow resistance.

7. The method of any one of Claims 1-6, further comprising:
receiving a medication identifier for the medication;
determining, based on medication identifier, a medication characteristic of the medication;
wherein the operating characteristic is also determined based on the identified medication characteristic.

8. The method of Claim 7, wherein the environmental condition comprises a temperature of an area proximate to the infusion pump or of the medication, and wherein the medication characteristic comprises a fluid viscosity at the temperature.

9. The method of any one of Claims 1-7, wherein the environmental condition comprises an ambient temperature or humidity of an area proximate to the infusion pump, a temperature of the medication, and wherein the operating characteristic of the infusion set is based on the temperature or humidity.

10. The method of any one of Claims 1-7, wherein the environmental condition comprises a circuit temperature associated with circuitry within the infusion pump.

11. The method of any one of Claims 1-10, further comprising:
identifying a threshold for providing an alarm regarding the administration of the medication by the infusion pump;
determining that operation of the infusion set under the monitored environmental condition affects how the threshold is satisfied for the administration of the medication; and
adjusting the pump speed or the flow rate to satisfy the threshold.

12. The method of any one of Claims 1-11, further comprising, after the administration of the medication has started:
measuring a changed delivery condition;
determining, based on the identification of the infusion set and the changed delivery condition, an updated operating characteristic of the infusion set corresponding to the changed delivery condition;
automatically adjusting the pump speed or the flow rate based on the updated operating characteristic of the infusion set corresponding to the changed delivery condition.

13. An infusion system, comprising:
a sensor; and
at least one processor and a non-transitory computer readable medium storing instructions that, when executed by the at least one processor, causes the at least one processor to perform a method according to any one of Claims 1-12, wherein the sensor is configured to monitor the environmental condition during or prior to the administration of the medication by the infusion pump.

14. The infusion system of Claim 13, further comprising:
the infusion pump, wherein the infusion pump comprises a housing including a fluid path to receive at least a portion of the infusion set,
wherein the sensor is disposed within the housing,
wherein the environmental condition is a condition within the housing of the infusion pump.

15. A non-transitory computer readable medium having instructions stored thereon that, when executed by a medical device, cause the medical device to perform a method according to any one of Claims 1-12.
